# EUROPÄISCHE PATENTSCHRIFT

(11) **EP 0 437 181 B1**
(45) Veröffentlichungstag und Bekanntmachung des Hinweises auf die Patenterteilung: **18.01.1995**
(21) Anmeldenummer: 90811022.4
(22) Anmeldetag: 21.12.1990
(51) Int. Cl.: A61N 5/06

(54) **Vorrichtung zum Bestrahlen der Bronchien eines Patienten für eine photodynamische Therapie**
Apparatus for irradiating the bronchia of a patient for a photodynamic therapy
Appareil pour irradier les bronches d'un patient pour une thérapie photodynamique

(30) Priorität: 09.01.1990 CH 58/90
(43) Veröffentlichungstag der Anmeldung: 17.07.1991
(73) Patentinhaber: CIBA-GEIGY AG, 4002 Basel (CH)
(72) Erfinder: Wagnières, Georges, CH-1095 Lutry (CH); Van den Bergh, Hubert, Dr., CH-1376 Goumoens-La-Ville (CH); Monnier, Philippe, Dr., CH-1010 Lausanne (CH)

(56) Entgegenhaltungen:
- WO-A-85/05262
- GB-A- 2 154 761
- US-A- 4 660 925

## Beschreibung

Die Erfindung betrifft eine Vorrichtung zum Bestrahlen der Bronchien eines Patienten für eine photodynamische Therapie mit einer das Licht eines Lasers in ein Bronchoskop einspeisenden optischen Faser.

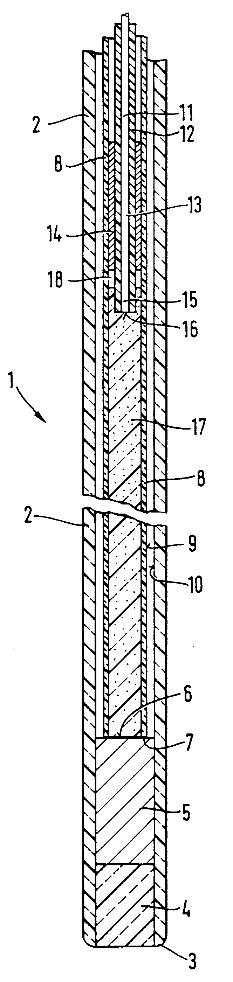

Ein derartiges Bronchoskop ist in Chemistry in Britain, Vol. 22, No. 5, May 1986, Hubert van den Bergh, "Light and porphyrins in cancer therapy" beschrieben und gestattet das Aufspüren und Behandeln von bösartigen Lungentumoren, insbesondere an den gabelförmigen Verzweigungen der Bronchien. Bei einer derartigen Behandlung wird einem Patienten Porphyrin injiziert. Nach mehreren Tagen hat das Tumorgewebe wesentlich mehr von diesem Farbstoff aufgenommen als das gesunde Gewebe. Bestrahlt man dann die verdächtige Stelle beispielsweise mit einem UV-Krypton-Laser bei ungefähr 410 nm, der an eine Quarzfaseroptik angeschlossen ist, so erkennt man das Krebsgewebe durch das von ihm ausgehende rote Licht. Neben diesem Effekt, der das Aufspüren von Tumoren ermöglicht, hat Porphyrin noch eine weitere vorteilhafte Eigenschaft, die darin besteht, dass es rotes Licht stark absorbiert, wobei in dem kranken Gewebe eine Reihe photochemischer Reaktionen ausgelöst wird, die das mit dem Porphyrin angereicherte Tumorgewebe abtöten. Das dazu benötigte rote Licht kann ebenfalls über eine Quarzfaseroptik zum Tumor geleitet werden, wodurch der Krebs bei einer derartigen photodynamischen Therapie selektiv zerstört wird. Die im Zusammenhang mit Fig. 3 der oben erwähnten Veröffentlichung beschriebene Vorrichtung gestattet es lediglich, einen vor dem Faserende liegenden Tumor zu bestrahlen. In den Fig. 8 und 9 ist eine Vorrichtung zum Bestrahlen des Oesophagus dargestellt, die es gestattet, das axial einfallende Licht radial zu diffundieren. Dazu wird eine mit einem Epoxidharz gefüllte Teflonröhre verwendet, wobei die Stirnfläche des Faserendes in einem axialen Abstand von der TiO₂-Partikel enthaltenden Epoxidharzmasse entfernt ist.

Eine weitere Vorrichtung zum Bestrahlen des Oesophagus ist aus der US-A-4,660,925 bekannt. Bei dieser Vorrichtung ist der am lichtaussendenden Ende freigelegte Faserkern in ein Streumedium eingebettet, welches seinerseits von einer tubusartigen lichtdurchlässigen Schutzhülle umgeben ist. Allerdings ragt das lichtaussendende Ende des Faserkerns sehr weit in das von der Schutzhülle umgebene Streumedium hinein, so dass die radiale Abstrahlung des Lichts auf einen im Verhältnis zu den Abmessungen der tubusartigen Schutzhüllen kleinen Bereich beschränkt ist. Ausserdem ist der Durchmesser dieser Schutzhülle erheblich grösser als der Durchmesser der Faser, so dass beim Durchführen dieser Vorrichtung durch den Biopsiekanal eines Bronchoskops Schwierigkeiten auftreten können, die durch die geometrischen Abmessungen dieser Vorrichtung bedingt sind.

Aus der WO-A-85/05262 ist eine medizinische und chirurgische Lasersonde bekannt. Diese umfasst eine optische Faser, die das Licht eines Lasers in eine in den Patientenkörper einzuführende Spitze einspeist. Die optische Faser ist von einem Lichtleiterschlauch umgeben, der die lichtaussendende Stirnfläche des Faserendes überragt. Sie ist mittels eines Montagezylinders in dem Lichtleiterschlauch fixiert. In diesem ist auch die Spitze fixiert, in die das aus der Faser austretende Licht eingespeist wird. Diese Spitze führt das Laserlicht zu dem zu bestrahelnden Gewebe.

Aus der GB-A-2,154,761 ist eine spitz zulaufende, das Licht streuende Endung einer optischen Faser bekannt, die besonders geeignet für die Behandlung von Tumoren mittels Bestrahlung mit Laserlicht ist. Die Spitze ist mit einem streuaktiven Material ummantelt. Die Spitze mitsamt dem auf ihr aufgebrachten streuaktiven Material wird dabei direkt in das Tumorgewebe eingeführt, ein Schutz für das streuaktive Material existiert nicht.

Ausgehend von diesem Stand der Technik liegt der Erfindung die Aufgabe zugrunde, eine Vorrichtung der eingangs genannten Art zu schaffen, die ausreichend klein ist, um in Bronchien vorgeführt werden zu können, und die auch nach längerer Verwendungsdauer das über die Faser eingespeiste Licht mit möglichst kleinen Verlusten radial diffundiert.

Diese Aufgabe wird durch die Merkmale im kennzeichnenden Teil des unabhängigen Patentanspruchs gelöst. Durch eine derartige Anordnung ergibt sich nicht nur die Möglichkeit einer Miniaturisierung, sondern wegen der hohen Transparenz von Silikon auch ein guter Wirkungsgrad sowie eine hohe Alterungsbeständigkeit, da das Silikon seine guten optischen Eigenschaften sehr lange beibehält. Das Faserende ist durch einen Zentrierzylinder im Lichtleiterschlauch fixiert. Zwischen diesem Zentrierzylinder und der Silikonmasse befindet sich in der Nähe der Stirnfläche ein um die optische Faser umlaufender radial durch den Lichtleiterschlauch begrenzter Ringspalt.

Der Lichtleiterschlauch besteht vorzugsweise aus PTFE und ist vorzugsweise von einem in axialer Richtung ihm gegenüber unverrückbaren Aussenschlauch umgeben, der über das distale Ende des Lichtleiterschlauches hinausragt, wobei in dem hinausragenden Abschnitt ein Aluminiumzylinder als Spiegel und ein PTFE-Zylinder als Abschlussstopfen angeordnet sind.

Zwischen dem Lichtleiterschlauch und dem Aussenschlauch ist ein freier Ringraum vorhanden. Der Lichtleiterschlauch steht etwa 3 cm über das Faserende über und bildet in diesem Abschnitt einen Diffusor. Dazu ist die Silikonmasse vorzugsweise mit 7 Promille TiO₂- Pulver mit einer Korngrösse von 0,2 Mikrometer vermischt.

Bei einem Ausführungsbeispiel der Erfindung hat der Lichtleiterschlauch eine lichte Weite von lediglich 0,8 mm und einen äusseren Durchmesser von etwa 1,1 mm. Der Aussenschlauch hat dann eine lichte Weite von 1,4 mm und einen Aussendurchmesser von 1,9 mm.

Nachfolgend wird ein Ausführungsbeispiel der Erfindung anhand der Zeichnung näher beschrieben. Die einzige Figur zeigt das vordere Ende einer in den Biopsiekanal eines Bronchoskopes einführbaren Vorrichtung gemäss der Erfindung.

In der Zeichnung erkennt man einen insgesamt mit dem Bezugszeichen 1 bezeichneten Bronchiallichtdiffusor. Der Bronchiallichtdiffusor 1 hat einen so geringen Durchmesser, dass er durch den Biopsiekanal eines Bronchoskopes hindurchpasst.

Der Bronchiallichtdiffusor 1 besteht aus einem Aussenschlauch 2 aus PTFE (Teflon) mit einer lichten Weite von 1,4 mm und einer Wandstärke von 0,25 mm Der Aussenschlauch 2 erstreckt sich ausgehend von dem in der Zeichnung unten dargestellten distalen Ende 3 bis zu einem Abstand, der um eine zweckmässige Länge grösser als die Länge des zu verwendenden Bronchoskopes ist. Am distalen Ende 3 ist der Aussenschlauch 2 durch einen Teflonstopfen 4 abgeschlossen, der mit seinem Aussenmantel dicht und fest mit dem Innenmantel des Aussenschlauches 2 verbunden ist.

Gegen die vom distalen Ende 3 wegweisenden Stirnseite des Teflonstopfens 4 liegt ein Aluminiumzylinder 5 an, dessen vom distalen Ende 3 wegweisende Stirnfläche 6 als Spiegel ausgebildet ist.

Gegen die Stirnfläche 6 liegt das distale Ende 7 eines Lichtleiterschlauches 8 aus PTFE an, der sich durch den Aussenschlauch 2 erstreckt, wobei zwischen dem Aussenmantel 9 des Lichtleiterschlauches 8 und dem Innenmantel 10 des Aussenschlauches 2 ein Ringspalt vorhanden ist. Der Lichtleiterschlauch 8 hat einen Aussendurchmesser von etwa 1,1 mm, während der Aussenschlauch 2 einen Innendurchmesser von 1,4 mm und einen Aussendurchmesser von 1,9 mm aufweist.

Wie man im oberen Teil der Zeichnung erkennt, ragt in den Lichtleiterschlauch 8 eine optische Faser 11 hinein, die mit einem Fasermantel 12 umgeben ist. Die optische Faser 11 hat einen Kerndurchmesser von 200 »m und einen Manteldurchmesser von 280 »m. Die numerische Apertur der optischen Faser beträgt vorzugsweise 0,21.

Das distale Ende 13 der optischen Faser 11 ist in einem Messing-Zentrierzylinder 14 befestigt, der seinerseits in axialer Richtung unverschiebbar im Lichtleiterschlauch 8 verklebt ist. Das Faserende 15 in unmittelbarer Nähe der lichtaussendenden Stirnfläche 16 ragt aus dem Zentrierzylinder 14 um ein kurzes Stück hervor und ist in eine Silikonmasse 17 in der Weise eingebettet, dass zwischen dem Zentrierzylinder 14 und der Silikonmasse 17 ein mit Luft erfüllter Ringspalt 18 vorhanden ist.

Die Silikonmasse 17 füllt den Raum zwischen der Stirnfläche 6 des Aluminiumzylinders 5 und dem Ringspalt 18 aus. Sie zeichnet sich durch eine hohe Transparenz aus und vergilbt auch im Laufe der Zeit nicht. Um das im wesentlichen axial aus der Stirnfläche 16 der optischen Faser 11 austretende Licht in radialer Richtung auf die Bronchialwände umzulenken, enthält die Silikonmasse Streupartikel. Diese bestehen aus einem Titanoxidpulver, das der Silikonmasse 17 beigemischt ist. Vorzugsweise enthält das Silikon 7 Promille TiO₂ mit einer Partikelgrösse von 0,2 »m.

Bei einem bevorzugten Ausführungsbeispiel ist die Konzentration der TiO₂-Partikel in der Silikonmasse 17 im Bereich der axialen Enden der den Lichtleiterschlauch 8 füllenden Silikonmasse 17 höher als im mittleren Bereich. Auf diese Weise wird in der Nähe der Stirnfläche 16 am Faserende 15 und in der Nähe der Stirnfläche 6 am Aluminiumzylinder 5 eine höhere Intensität des dort radial gestreuten Lichtes bewirkt.

Auf diese Weise entsteht ein Licht über 360 Grad radial aussendender Bronchiallichtdiffusor mit einer Länge von etwa 3 cm, der aufgrund seines geringen Durchmessers auch in sehr enge Bronchien vorgeführt werden kann.

Wenn statt einer Lichtaussendung über 360 Grad ein Bestrahlungswinkel von z. B. 180 Grad gewünscht ist, kann die Innenseite des Aussenschlauches 2 mit einer Verspiegelung versehen sein, die sich rinnenförmig über den halben Mantelumfang erstreckt.

## Patentansprüche

1. Vorrichtung zum Bestrahlen der Bronchien eines Patienten für eine photodynamische Therapie mit einer das Licht eines Lasers in ein Bronchoskop einspeisenden optischen Faser (11), welche von einem Lichtleiterschlauch (8) umgeben ist, der die lichtaussendende Stirnfläche (6) des Faserendes (15) um ein Vielfaches seines Durchmessers übertagt, wobei das Faserende (15) durch einen Zentrierzylinder (14) im Lichtleiterschlauch (8) fixiert ist, dadurch gekennzeichnet, dass das Faserende (15) in einer TiO₂-Pulver enthaltenden Silikonmasse (17) eingebettet ist, die sich ausgehend vom Faserende (15) bis zum Lichtleiterschlauchende (7) erstreckt, und dass zwischen dem Zentrierzylinder (14) und der Silikonmasse (17) ein in der Nähe der Stirnfläche (16) um die optische Faser (11) umlaufender radial durch den Lichtleiterschlauch (8) begrenzter Ringspalt (18) vorgesehen ist.

2. Vorrichtung nach Anspruch 1, dadurch gekennzeichnet, dass der Lichtleiterschlauch (8) aus PTFE besteht.

3. Vorrichtung nach einem der vorstehenden Ansprüche, dadurch gekennzeichnet, dass der Lichtleiterschlauch (8) von einem in axialer Richtung unverrückbaren Aussenschlauch (2) umgeben ist, der über das distale Ende (7) des Lichtleiterschlauches (8) hinausragt, und dass in dem hinausragenden Abschnitt ein Aluminiumzylinder (5) als Spiegel und ein PTFE Zylinder (4) als Abschlussstopfen angeordnet sind.

4. Vorrichtung nach Anspruch 3, dadurch gekennzeichnet, dass zwischen dem Lichtleiterschlauch (8) und dem Aussenschlauch (2) ein freier Ringraum vorhanden ist.

5. Vorrichtung nach einem der vorstehenden Ansprüche, dadurch gekennzeichnet, dass der Lichtleiterschlauch (8) etwa drei cm über das Faserende (13, 15, 16) übersteht.

6. Vorrichtung nach einem der vorstehenden Ansprüche, dadurch gekennzeichnet, dass die Silikonmasse (17) sieben Promille TiO₂-Pulver mit einer Korngrösse von 0,2 »m enthält.

7. Vorrichtung nach einem der vorstehenden Ansprüche, dadurch gekennzeichnet, dass der Lichtleiterschlauch (8) eine lichte Weite von 0,8 mm und einen äusseren Durchmesser von etwa 1,1 mm hat.

8. Vorrichtung nach Anspruch 7, dadurch gekennzeichnet, dass der Aussenschlauch (2) aus PTFE besteht und eine lichte Weite von 1,4 mm und einen Aussendurchmesser von 1,9 mm hat.

9. Vorrichtung nach Anspruch 6, dadurch gekennzeichnet, dass die Konzentration des TiO₂-Pulvers im axial mittleren Bereich kleiner als in der Nähe der Stirnflächen (6, 16) der optischen Faser (11) und des Aluminiumzylinders (5) ist.

## Claims

1. An apparatus for irradiating the bronchi of a patient for the purpose of photodynamic therapy, which apparatus has an optical fibre (11) that feeds the light of a laser into a bronchoscope and is surrounded by a light-guide tube (8) which projects beyond the light-emitting face (6) of the end (15) of the fibre by many times the fibre's diameter, the end (15) of the fibre being fixed in position in the light-guide tube (8) by a centring cylinder (14), wherein the end (15) of the fibre is embedded in a silicone composition (17) that comprises TiO₂ powder and that extends from the end (15) of the fibre to the end (7) of the light-guide tube, and an annular gap (18) that extends round the optical fibre (11) in the vicinity of the end face (16) and is delimited radially by the light-guide tube (8) is provided between the centring cylinder (14) and the silicone composition (17).

2. An apparatus according to claim 1, wherein the light-guide tube (8) consists of PTFE.

3. An apparatus according to any one of the preceding claims, wherein the light-guide tube (8) is surrounded by an outer tube (2) that is immovable in the axial direction and that projects beyond the distal end (7) of the light-guide tube (8), and an aluminium cylinder (5) which acts as a mirror and a PTFE cylinder (4) which acts as a closing plug are arranged in the projecting portion.

4. An apparatus according to claim 3, wherein an open annular space is present between the light-guide tube (8) and the outer tube (2).

5. An apparatus according to any one of the preceding claims, wherein the light-guide tube (8) projects by approximately 3 cm beyond the end (13, 15, 16) of the fibre.

6. An apparatus according to any one of the preceding claims, wherein the silicone composition (17) comprises seven parts per thousand of TiO₂ powder having a particle size of 0.2 »m.

7. An apparatus according to any one of the preceding claims, wherein the light-guide tube (8) has an inside diameter of 0.8 mm and an outside diameter of approximately 1.1 mm.

8. An apparatus according to claim 7, wherein the outer tube (2) consists of PTFE and has an inside diameter of 1.4 mm and an outside diameter of 1.9 mm.

9. An apparatus according to claim 6, wherein the concentration of the TiO₂ powder is lower in the axially central region than it is in the vicinity of the end faces (6, 16) of the optical fibre (11) and the aluminium cylinder (5).

## Revendications

1. Appareil pour irradier les bronches d'un malade aux fins d'une thérapie photodynamique, comprenant une fibre optique (11) injectant la lumière d'un laser dans un bronchoscope, qui est entourée d'un tuyau souple (8) conducteur de lumière dépassant de la face terminale (16) émettrice de lumière de l'extrémité (15) de la fibre sur une longueur correspondant à un multiple de son diamètre, l'extrémité (15) de la fibre étant fixée par un cylindre de centrage (14) dans le tuyau conducteur de lumière (8),appareil caractérisé en ce que l'extrémité (15) de la fibre est noyée dans une masse de silicone (17) contenant de la poudre de TiO₂ et s'étendant à partir de l'extrémité (15) de la fibre jusqu'à l'extrémité (7) du tuyau conducteur de lumière, et qu'une fente annulaire (18), entourant la fibre optique (11) et délimitée radialement par le tuyau (8) conducteur de lumière, est prévue entre le cylindre de centrage (14) et la masse de silicone (17), à proximité de la face terminale (16) de la fibre.

2. Appareil selon la revendication 1, caractérisé en ce que le tuyau souple (8) conducteur de lumière est en polytétrafluoroéthylène (PTFE).

3. Appareil selon une des revendications précédentes, caractérisé en ce que le tuyau souple (8) conducteur de lumière est entouré d'un tuyau souple extérieur (2) axialement immobile et dépassant de l'extrémité distale (7) du tuyau (8) conducteur de lumière et qu'un cylindre d'aluminium (5) servant de miroir et un cylindre (4) en PTFE, servant de bouchon de fermeture, sont disposés dans la partie saillante du tuyau extérieur (2).

4. Appareil selon la revendication 3, caractérisé en ce qu'un espace annulaire libre est prévu entre le tuyau souple (8) conducteur de lumière et le tuyau souple extérieur (2).

5. Appareil selon une des revendications précédentes, caractérisé en ce que le tuyau (8) conducteur de lumière dépasse d'environ trois cm de l'extrémité (13, 15, 16) de la fibre.

6. Appareil selon une des revendications précédentes, caractérisé en ce que la masse de silicone (17) contient sept pour-mille de poudre de TiO₂ d'une grosseur de grain de 0,2 »m.

7. Appareil selon une des revendications précédentes, caractérisé en ce que le tuyau (8) conducteur de lumière possède un diamètre intérieur de 0,8 mm et un diamètre extérieur d'environ 1,1 mm.

8. Appareil selon la revendication 7, caractérisé en ce que le tuyau extérieur (2) est en PTFE et possède un diamètre intérieur de 1,4 mm et un diamètre extérieur de 1,9 mm.

9. Appareil selon la revendication 6, caractérisé en ce que la concentration de la poudre de TiO₂ est plus petite dans la zone axialement centrale qu'à proximité des faces terminales (6, 16) de la fibre optique (11) et du cylindre d'aluminium (5) respectivement.
